# EUROPEAN PATENT APPLICATION

(11) **EP 1 782 737 A1**
(43) Date of publication of application: **09.05.2007**
(21) Application number: 06076973.4
(22) Date of filing: 01.11.2006
(51) Int. Cl.: A61B 10/00, G01N 1/31, G01N 1/36, B01L 3/00

(54) **Tissue cassette for transporting**

(30) Priority: 02.11.2005 US 732549 P
(71) Applicant: BioPath Automation, L.L.C., Loveland, Ohio 45140 (US)
(72) Inventor: Williamson, Warren P., IV, Loveland, Ohio 45140 (US)
(74) Representative: Findlay, Alice Rosemary

(57) **Abstract**

A cassette (100') for transporting tissue including a first flat reference porous structure (116') for supporting the tissue, and a second porous structure (128') having a compression resistance. The first flat reference porous structure (116') and the second porous structure (128') can be positioned into an abutting, contacting or non-contacting position for securing the tissue therebetween.

## Description

The present invention relates to devices for the transport of tissue from a harvesting site to a pathology lab, more particularly, but not exclusively, to a tissue cassette for transporting tissue from a harvesting site to a pathology lab.

Screening tissue samples for disease is an extremely common practice in modern medicine. Otherwise known as a biopsy, a patient has tissue samples harvested from their body by a physician or other medical professional and then transported to a pathology lab in a container filled with tissue preservative for slide preparation, review, and diagnosis. When tissue is deposited unrestrained into tissue preservative solution it can curl and contort as it hardens. Some examples of tissue types that are prone to curl are colon tissue and skin tissue, however, other types of tissue curl or distort as well.

More specifically, after the physician or other medical professional has harvested the tissue and obtained the biopsy sample, the biopsy sample is then placed into what is known as a "fixing solution" preservative solution. The fixing or preservative solution is commonly a solution of buffered formaldehyde known as formalin. For example, a biopsy sample is commonly harvested by using a sharp, hollow needle to gather tissue inside the lumen of the needle. Accordingly, the biopsy samples are commonly long and skinny and rather snake-like. The preservative solution will kill the pathogens to protect the safety of the pathology lab workers. The tissue can curl up into a ball or take on other three dimensional contortions. Therefore, by the time the biopsy samples have arrived at the pathology lab, they may have hardened or semi-hardened into contorted shapes.

The tissue samples must be embedded in a paraffin block for sectioning and subsequent diagnosis. The biopsy samples must be reconfigured to be perfectly flat in the paraffin mold. Any contorted or curled biopsy sample must be straightened before embedding in the paraffin. Without straightening the tissue sample, a misdiagnosis could occur for reasons to be discussed below. Straightening these biopsy samples is difficult because a high level of precision is necessary and the size of the sample is often extremely small.

After the sample has been embedded, a microtome is used to slice very thin sections of the biopsy sample and paraffin combination. The average section is usually 3 Φm to 5 Φm thick. Usually, the technologist will take no more than about thirty slices into the biopsy and paraffin combination. The total depth into the paraffin of all of these combined sections is around 0.001 inch. Therefore, if the biopsy sample is not correctly repositioned to be perfectly flat before it is embedded in the paraffin, it is quite possible that a portion of the biopsy sample will never be sectioned and thus excluded from the pathologist's subsequent examination.

Many times, landmark indicators are used when taking biopsy samples. Landmark indicators indicate to the medical professional the location of harvest relative to the patient's body. The landmark indicators ensure that a follow-up can be accurately planned during a subsequent surgery, in staging of the tumor, etc. Sutures have been secured to the biopsy sample to provide one type of landmark indicator. However, sutures can be difficult and time consuming to apply. Currently, there is a need for a less time-consuming and more accurate manner to identify the orientation of the sample in relation to the patient's anatomy.

One aspect of the invention is a cassette for holding tissue. The cassette includes a first flat reference porous structure for supporting the tissue. The cassette also includes a second porous structure having a compression resistance. The first flat reference porous structure and the second porous structure can be positioned into an abutting position for securing the tissue therebetween.

Another aspect of the invention is a method for improving the quality of sections of a biopsy sample. The method includes placing the biopsy sample into a tissue cassette and moving at least a portion of the tissue cassette to apply a compressive force to flatten the biopsy sample against a flat reference surface of the tissue cassette.

The invention will now be described by way of example with reference to the accompanying drawings in which:
FIG. 1 is a perspective view of a tissue cassette in an open position according to one embodiment.
FIG. 2 is a cross-sectional view of the tissue cassette taken generally along line 2-2 in FIG. 1 but shown in a closed or latched position.
FIG. 3 is a perspective cross-sectional view of the tissue cassette also taken generally along line 2-2 of FIG. 1 and illustrating the cassette in an open position.
FIGS. 4A-C are top plan views of a landmark indication system in different uses with the tissue cassette of FIG. 1.
FIGS. 5A and 5B are illustrations of respective first and second microscope slides comparing biopsy samples that had been previously held in a conventional biopsy container (FIG. 5A) and samples held with the tissue cassette of FIG. 1 (FIG. 5B).
FIGS. 6A and 6B are further illustrations of respective first and second microscope slides comparing biopsy samples that had been previously held in a conventional biopsy container with formalin (FIG. 6A) and samples held with the tissue cassette of Fig. 1 (FIG. 6B).
FIG. 7A illustrates a tissue sample obtained from a conventional tissue transporting or biopsy container, with the tissue positioned inside of a paraffin mold prior to embedding and sectioning.
FIG. 7B illustrates a section of the tissue sample of FIG. 7A positioned upon a slide for diagnosis by a medical professional.
FIG. 8A illustrates a tissue sample obtained from the tissue cassette of FIG. 1, with the tissue positioned inside of a paraffin mold prior to embedding and sectioning.
FIG. 8B illustrates a section of the tissue sample of FIG. 8A positioned upon a slide for diagnosis by a medical professional.
FIG. 9 is a perspective view of a tissue cassette according to another embodiment and shown in a closed position.
FIG. 10 is a perspective view of the tissue cassette shown in FIG. 9, but illustrated prior to the securement of respective porous membranes.
FIG. 11 is a cross sectional view taken along line 11-11 of FIG. 9.
FIG. 11A is an enlarged view of encircled portion 11A shown in FIG. 11.

The present application is directed to a cassette for transporting tissue after a biopsy has been performed. Generally, the cassette includes two opposing porous surfaces for fixing or holding tissue samples therebetween.

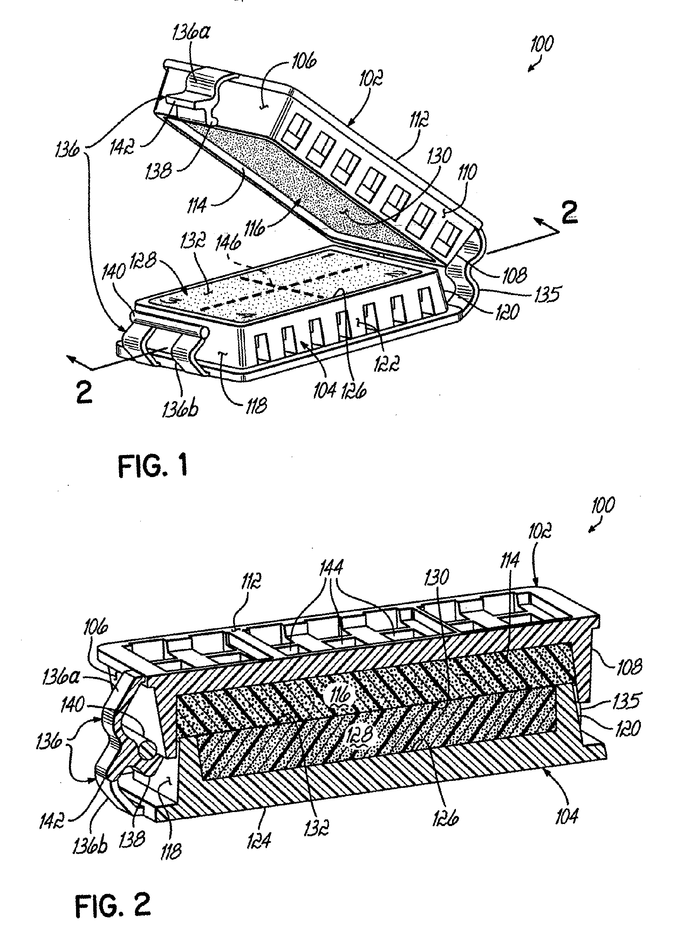

Referring now to the drawings, FIG. 1 illustrates a tissue cassette 100. The tissue cassette 100 is constructed and arranged to hold biopsy tissue samples after they have been removed from the patient and before the pathologist processes them for inspection. The tissue cassette 100 is therefore designed to be placed in another container (not shown) adapted to hold a tissue preservative solution, such as a formalin solution, such that the solution can fully contact the tissue cassette 100 and any tissue sample(s) therein. The tissue cassette 100 includes a first perforated structure or frame 102 and a second perforated structure or frame 104. The terms "perforated" and "porous" are used herein as analogous or synonymous terms meant to convey the fact that fluid solution can reach the tissue through the pores or perforations of the structure. As illustrated in FIG. 1, the first perforated structure 102 and the second perforated structure 104 are generally of the same size and are positioned opposite from one another. This arrangement allows the first perforated structure 102 and the second perforated structure 104 to easily be brought together to apply a compressive force to the biopsy sample after it has been inserted therein. The first perforated structure 102 includes a forward surface 106, a rearward surface 108, and two side surfaces 110. It also includes an outer surface 112. The rearward surface 108, and any other surface, can have multiple minor surfaces that taken together comprise the surface. These five surfaces 106, 108, 110, and 112 combine together to define an interior area 114 that contains a first porous structure 116. In some embodiments, the porous structure 116 is a foam pad, however, in other embodiments other types of materials may be used. Some types of materials for the foam pad are polyester or polyurethane with an open cell reticulated structure. Furthermore, special foams with hydrophilic properties could be used to assure wetting of intricate samples. In addition, while one porous structure 116 is illustrated inside the interior area 114 in FIG. 1, two or more porous structures can be used in other embodiments. In this illustrated embodiment, the interior area 114 is substantially box shaped, however, in other embodiments the first perforated structure 102 may have an interior area 114 that is different in configuration. For example, the interior area 114 could be an oval shape, a cylindrical shape, a rectangular shape, a square shape, or any other area readily apparent to those skilled in this art. Accordingly, the interior area 114 may be sized to receive a variety of biopsy sample sizes.

Similarly, the second perforated structure or frame 104 includes a forward surface 118, a rearward surface 120, side surfaces 122, and an outer surface 124. The combination of all these surfaces 118, 120, 122, and 124 defines an interior area 126 that contains a second porous structure 128 that may be constructed out of foam or other materials. The perforated structures 102, 104 can be constructed out of a variety of materials. In the illustrated embodiment, the perforated structures 102, 104 are constructed out of a plastic material, such as a high-density polyethylene (HDPE) or Acetel. In other embodiments, however, other materials may be used. In addition, the porous structures 116, 128 may include porous membranes 130 and 132. These porous membranes 130, 132 are constructed and arranged to avoid damage to the tissue sample(s) and to minimize or prevent artifacts in the tissue sample(s) so extremely small biopsy samples, such as those less than 0.5 mm in size, can be effectively secured in the tissue cassette 100. In the illustrated embodiment, the porous membranes 130, 132 may be formed out of lens paper or filter paper, however, those skilled in the art will recognize that other materials can be used in other embodiments. For example, the porous membranes 130, 132 can be a porous material such as a thermoplastic porous film or netting, a woven or non-woven material made from cotton, or other natural or synthetic fiber materials or other suitable materials. One suitable material is sold by Delstar Technologies, Inc., Middletown, Delaware, under the name Delnet® and is an apertured film or netting formed from high density polyethylene, having a flat surface facing the tissue samples. One or both membranes 130, 132 may be eliminated as long as undesirable artifacts are not formed on the biopsy sample(s) by the porous structures 116, 128. The membranes 130, 132 may be about 0.001 inch thick to allow unimpeded fixing fluid access and wicking action to the tissue surface. In addition, the membranes 130, 132 have a porosity of about 100 µm to about 400 µm some having porosity closer to about 200 µm. The membranes 130, 132 should remain taut and should remain temperature, moisture, and reagent stable. In addition, the membranes 130, 132 should not degrade when placed in the reagent solution, such as a formalin solution, and may be designed or formulated so as not to degrade when exposed to the chemicals used in the tissue processing. Furthermore, the membranes 130, 132 may be heat staked to the porous structures 116, 128 or the perforated structures 102, 104 or may be fixed in place by any other suitable method. The membranes 130, 132 need not be the same material. For instance, one of the membranes 130, 132 can be thinner and more compliant and conform to undulations in the biopsy sample. In addition, one of the membranes 130, 132 and the corresponding porous structure 116, 128 may be simply trapped in its perforated structure 102, 104 thereby allowing a free-floating configuration particularly adapted to conform to thick and thin tissue if necessary. In sum, these artifact inhibiting or minimizing porous membranes 130, 132 are sufficiently compliant to avoid damaging the biopsy sample, but sufficiently firm for flattening the biopsy sample before embedding in a material, such as paraffin.

The porous structures 116, 128 may have differing levels of compression resistance. Some embodiments, however, have porous structures 116, 128 that have the same level of compression resistance. Typically, the compression resistance ranges between about 0.5 lbs/in² to about 4 lbs/in² at about 50% compression. One porous structure 116 or 128 may be more compliant and have a compression resistance of about 0.5 Ibs/in². The other porous structure 116 or 128 may be less compliant with a compression resistance of about 2-4 lbs/in². The porous structure 116 or 128 having the higher compression resistance can also be known as the reference structure and its corresponding porous membrane 130, 132 can be known as the reference surface. The typical porosity of the porous structures 116, 128 is around 0.020" to 0.025" open cell pores. In addition, in some embodiments, polyurethane foam with a hydrophilic-formulation can be used without a porous membrane if the wetting is great enough and the pore size is small enough. Moreover, the porous structures 116, 128 should be formed out of a material that does not degrade in the formalin or, in some cases, the chemicals used in the tissue processing. The smaller pore size would trap the biopsy sample in the tissue cassette 100 and the enhanced wetting properties would overcome the potential disadvantage of the smaller pore size.

In the illustrated embodiment, the second porous structure 128 has more compression resistance than the first porous structure 116. Accordingly, when a biopsy sample is introduced into the tissue cassette 100, the porous structure 128 having higher compression resistance will provide resistance to deformation upon closing of the tissue cassette 100. Therefore, the flat surface of the biopsy sample will be created along the surface of the biopsy sample that is in contact with the porous membrane 132 and the second porous structure 128.

The tissue cassette 100 also includes a connector system made up of a compliant hinge 135 and a clasp 136. Those skilled in the art, however, recognize that other connector systems can be used in other embodiments. For example, in one alternative embodiment, the connector system could be two clasps that lock together on either side of the tissue cassette 100. Moreover, in another embodiment, the connector system 134 could be elastic bands that wrap around the periphery of the perforated structures 102, 104. Accordingly, any structure that can be used to urge the porous structures 116, 128 together is contemplated. In addition, not all tissue cassettes require a connector because the resilient porous structures 116, 128 could apply sufficient force in some other manner not requiring a connector.

The compliant hinge 135 is coupled to the first perforated structure 102 at the rearward surface 108. The compliant hinge 135 is also coupled to the second perforated structure 104 at the rearward surface 120. This arrangement provides a "clam shell" like design that allows the first perforated structure 102 and the second perforated structure 104 to be easily separated from one another and to easily clamp down. Moreover, this arrangement allows for a maximum separation between the forward end surfaces 106 and 118 of the first and second perforated structures 102 and 104. This arrangement enables a user to easily place the biopsy sample inside the interior areas 114 and 126 using the harvesting instrument, and, if necessary, a portion of the medical professional's hand.

The clasp 136 illustrated in FIG. 1 is one structure that can assist in coupling the perforated structures 102, 104, however, those skilled in the art recognize that other mechanisms can be used so long as they provide for a compressive force to be applied to the biopsy sample upon closure. The clasp 136 of the illustrated embodiment has a top portion 136a and a bottom portion 136b. The top portion 136a provides a latch 138 at the end of the top portion 136a. This latch 138 slides over a bar 140 located on the bottom portion 136b, and then retracts around the bar 140 to firmly lock into place. In addition, the top portion 136a includes a tab 142 which allows the medical professional to easily open the tissue cassette 100 when needed by simply pressing their thumb or finger to the underside of the tab 142 in order to retract the latch 138 from underneath the bar 140. The clasp 136 forces the opposing porous surfaces 116, 128 together so as to apply a compressive force to the biopsy sample that is placed therein. The clasp 136 also includes the latch 138 and bar 140 to maintain that compressive force constantly and uniformly until the tissue cassette 100 is opened and the biopsy sample is ready to be embedded in a material, such as paraffin.

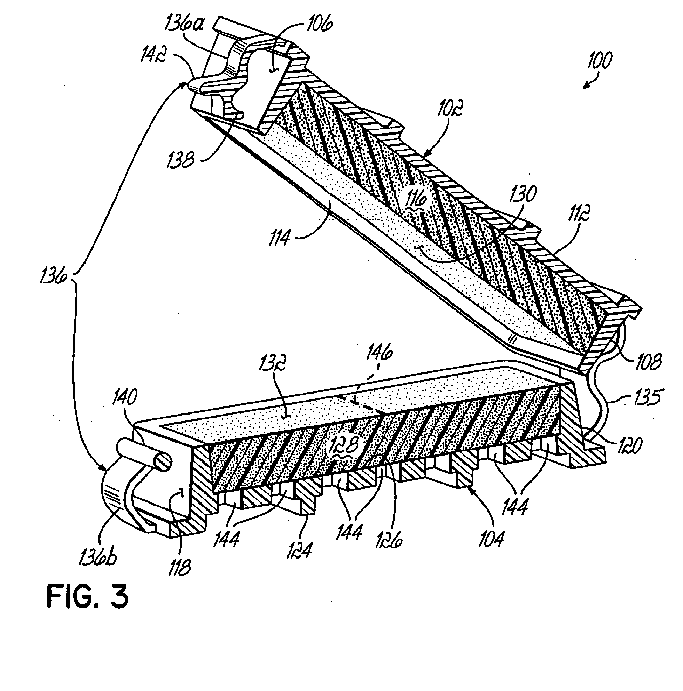

FIGS. 2 and 3 respectively illustrate the biopsy cassette 100 in closed and open positions. Outer surface 112 is shown to have a plurality of pores 144. These pores 144 allow for introduction of the fixing solution, such as formalin. The porous structures 116, 128 allow the fluid to reach the porous membranes 130, 132 so that the biopsy sample can become fixed in its flattened and undamaged state. Therefore, the tissue cassette 100 prepares the biopsy sample for inspection.

In use, the tissue cassette 100 operates to properly flatten and leave undamaged a biopsy sample placed therein. Initially, a medical professional performs a biopsy on a patient to obtain a biopsy sample. This is usually done using a needle or other hollow instrument in order to obtain a biopsy sample inside of the lumen defined in the needle or other instrument. The biopsy sample is then taken directly to the tissue cassette 100 where it is placed on one of the porous membranes 130, 132. At this point, the biopsy sample may not be flat. It could be coiled or contorted in any number of configurations.

The tissue cassette 100 is then closed and the porous membranes 130, 132 may apply uniform and constant pressure to the biopsy sample. The differences in compression resistance between the porous structures 116, 128 result in one of the sides of the biopsy sample becoming flattened. The second porous structure 128 having a higher compression resistance will not compress substantially and the first porous structure 116 will compress so as not to damage the biopsy sample and introduce artifact therein. The first porous structure 116 may surround all sides of the tissue sample except the side which is against the second porous structure 128. Flattening of one side of the tissue sample can occur due to one of the porous membranes 130 or 132 being taut. In addition, the other porous membrane 130 or 132 can be free-floating inside of its perforated structure 102, 104, either by not being heat staked and simply resting upon a porous structure 116 or 128 or by being fixed only to the porous structure 116 or 128 and not fixed to the perforated structure 102, 104. Likewise, the porous structure 116 and/or 128 may or may not be fixed to the associated perforated structure 102, 104. Accordingly, the biopsy sample may be flattened and fixed to prevent curling or distortion of the biopsy sample and a loss of visible margins.

Tissue cassette 100 may be immersed in a container filled with a fixing solution. The biopsy sample can then be hardened with one side flat and without visible artifact. Having a flat surface can be especially important for skin biopsy samples. The tissue cassette 100 can thereafter be stored until it is ready to be opened by a medical professional. Use of tissue cassettes 100 may allow a biopsy sample to be held flat, for example, within less than a 0.0025 inch variance. This level of precision can be important for skin tissue biopsies because the samples do not curl up and distort the margins for excision of malignant tissue. Therefore, when the technologist introduces the biopsy sample into the paraffin and then makes slices using a microtome, the possibility that an incorrect diagnosis due to curling or distortion of the biopsy sample is reduced because the margin intended by the surgeon or other medical professional taking the sample is properly preserved throughout the histological process. The pathologist has the assurance that the margin that will be delivered back to the harvesting medical professional will be as the medical professional intended.

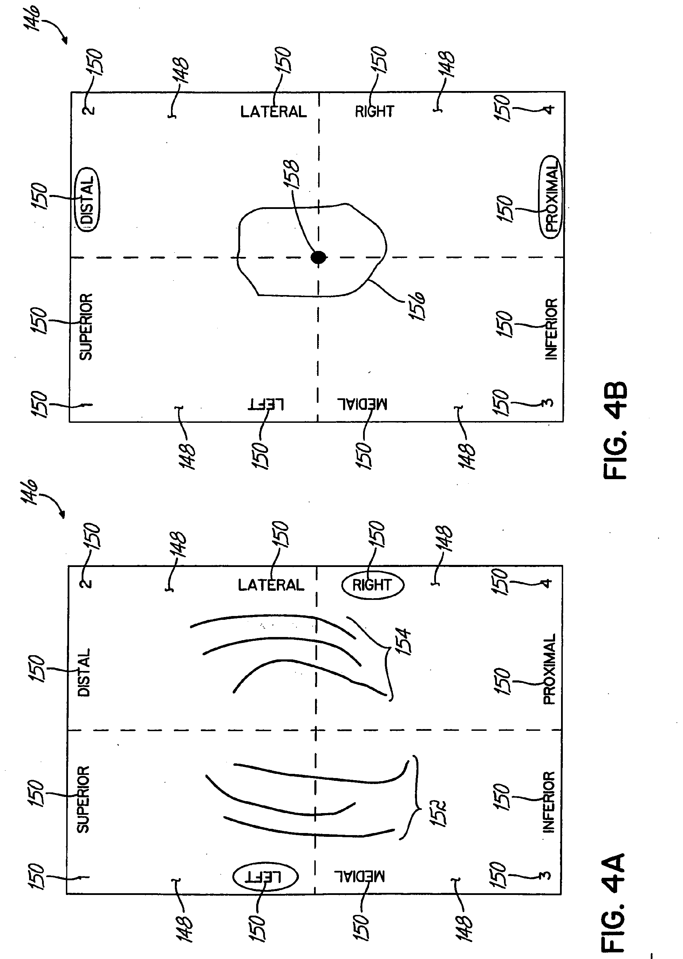

Referring now to FIGS. 4A-C, a landmark indication system 146 may be used on, for example, the porous membrane 132 associated with the porous structure 128 of greater compression resistance. The medical professional that harvests the biopsy sample places the biopsy sample on the porous membrane 132 and uses the landmark indication system 146 to communicate the anatomic position of the harvested tissue or other information concerning the biopsy sample. The landmark indication system 146 includes four quadrants 148 that are identified using labels 150. Illustrative examples are provided in FIGS. 4A-C discussed below. FIGS. 4A-C illustrate some uses of the landmark indication system 146, however, those skilled in this art recognize that the landmark indication system 146 can be used in other manners.

Referring now to FIG. 4A, the landmark indication system 146 is used with biopsy samples 152 and 154 taken from a prostate gland. The biopsy samples 152 and 154 represent prostate cores harvested by a medical professional during a biopsy. Biopsy samples 152 illustrate cores harvested from the left side of the prostate gland. Accordingly, the "Left" label 150 is circled to make this indication. Similarly, the biopsy samples 154 are taken from the right side of the prostate gland and the "Right" label 150 is accordingly circled. Thus, the histotechnician or other medical profession will be able to label the paraffin sections to accurately reflect the area of the prostate where the cores were harvested.

FIG. 4B illustrates another use of the landmark indication system 146. In the center of the landmark indication system 146 is a skin tissue sample 156 taken from a finger. The skin tissue sample 156 includes a lesion 158. The orientation of the skin tissue sample 156 is identified by circling the "Proximal" and "Distal" labels 150 as illustrated in FIG. 4B. Again, this system enables the medical professional harvesting the skin tissue sample 156 to easily communicate the orientation of the skin sample, relative to the proximal and distal ends of the patient's finger. Thus, the likelihood of error decreases.

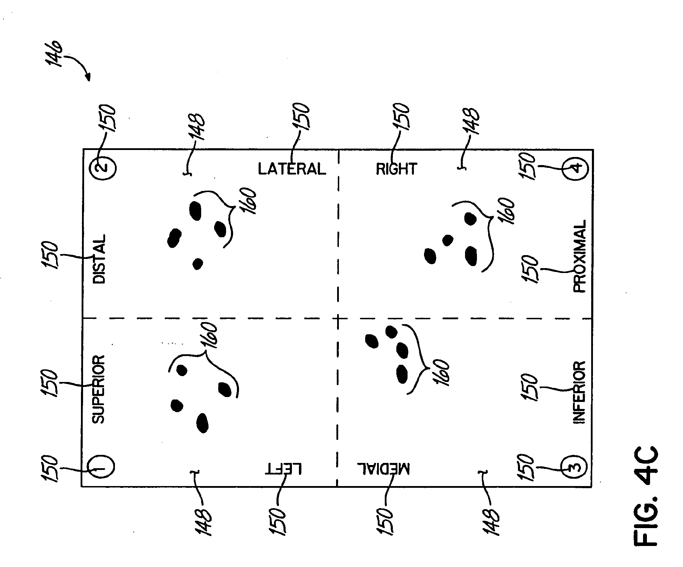

FIG. 4C illustrates the landmark indication system 146 for use with taking a multitude of biopsy samples 160. The tissue cassette 100 will keep the biopsy samples 160 in place once it is closed. Thus, the medical professional, such as a surgeon, can create a surgical report and make notes under the heading of quadrant "1" corresponding to the label 150 of "1" that is circled. Such notes can describe characteristics of those samples. For instance, assume that the samples 160 in the quadrant 148 having the "1" label 150 are all from the pancreas. In addition, assume a biopsy was also taken from the gall bladder, kidney, and liver. The samples 160 can be organized in the quadrants 148 and then the labels 150 corresponding to the numbers can be circled. The surgical report can be written by the surgeon to provide information noting that the samples 160 in the quadrant 148 with the label 150 having "1" were taken from the pancreas, those under "2" from the gall bladder, and so forth. Thus, the landmark indication system 146 can provide information to the pathologist or histotechnician in many different manners.

The pathologist and the histotechnician can use information from labels 150 communicated by the medical professional when subsequently preparing the gross description. The gross description is prepared by the histotechnician or the pathologist opening the tissue cassette 100 and observing the number, placement, size, and/or anatomic orientation indicated by the medical professional who harvested the tissue. Subsequently, the tissue cassette 100 can be closed for further tissue processing without the need for additional manipulation of the biopsy sample before embedding. The tissue cassette 100 completely preserves the orientation of the biopsy sample throughout the entire tissue fixing process.

To perform an embedding process the histotechnician or the pathologist removes the biopsy sample from the tissue cassette 100. The landmark indication system 146 makes the anatomic harvest position easily identifiable and able to be oriented and processed into standard embedding molds. Alternatively, when using a system having a sectionable cassette, the tissue cassette 100 keeps the biopsy sample flat and indicates the harvested orientation of the biopsy samples using the landmark indicator system 146. The tissue is prefixed and substantially hardened before removing it from the tissue cassette 100 and placing it into a sectionable cassette, other support, embedding medium mold, etc.

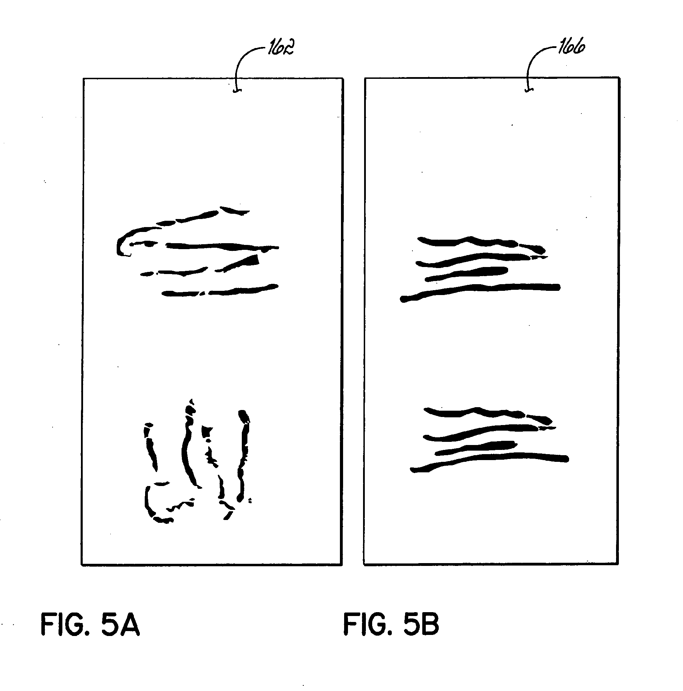

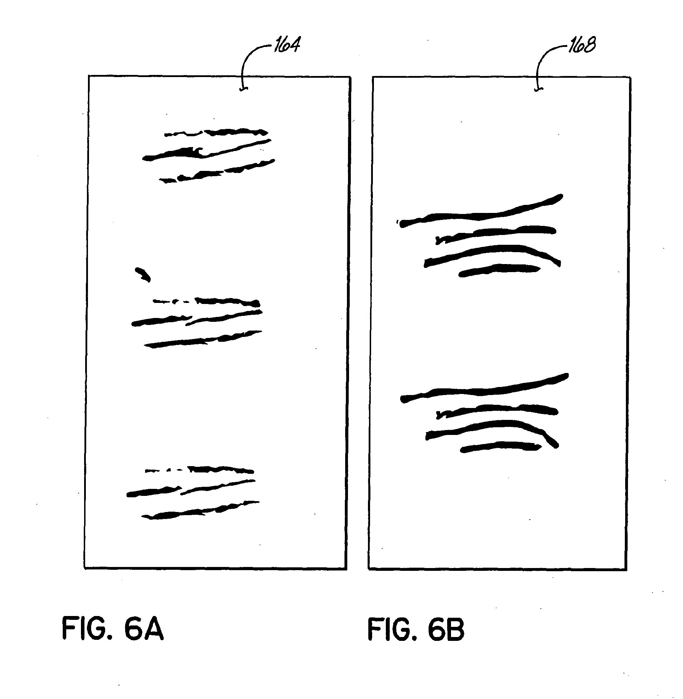

Referring now to FIGS. 5A and 5B, and FIGS. 6A and 6B, first slides 162, 164 shown respectively in FIGS. 5A and 6A illustrate biopsy samples taken from a conventional tissue preservative container and freely submerged in the preservative solution. The second slides 166, 168 shown respectively in FIGS. 5B and 6B illustrate biopsy samples taken from the tissue cassette 100 which was then submerged in the preservative solution. The second slides 166, 168 illustrate samples that are defined and have full width. The robustness of the sections of the slide is improved. Accordingly, the tissue cassette 100 greatly reduces the margin of error during embedding a biopsy sample for later diagnosis.

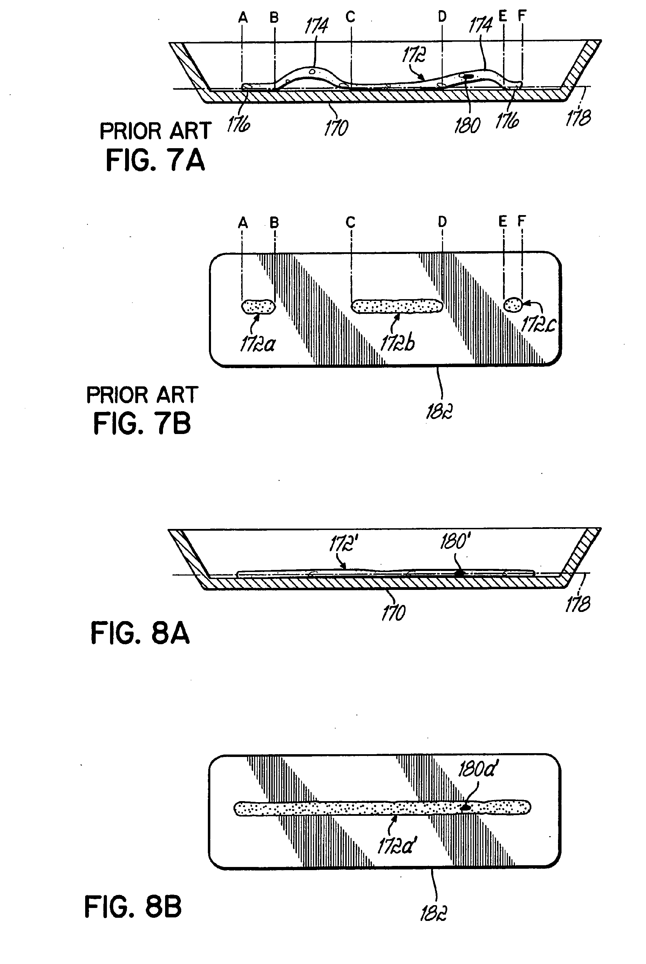

FIG. 7A illustrates a mold 170 for holding a biopsy sample 172 during embedding with a material such as paraffin wax. The mold 170 receives the biopsy sample 172 after sample 172 has hardened into the configuration illustrated. The biopsy sample 172 is convoluted and includes high points 174 and low points 176. After embedding the biopsy sample 172 in paraffin wax, a plurality of sections can be taken through the biopsy sample 172 using a microtome. The line 178 illustrates the most common area that a section will be taken through the biopsy sample 172. The biopsy sample 172 includes a lesion 180, such as a group of cancerous cells. The line 178 is below the lesion 180. Referring to FIG. 7B, the section taken through line 178 is illustrated as being placed upon a slide 182. Three sections of the tissue sample 172 are located on the slide 182. The first section 172a corresponds to the length of the biopsy sample 172 between points A and B illustrated in FIGS. 7A and 7B. The second section 172b corresponds to the length of the biopsy sample 172 between points C and D. The third section 172c corresponds to the length of the biopsy sample between points E and F. The lesion 180 does not appear because it is between points D and E close to a high point 174. Accordingly, the diagnosis will be inaccurate.

Referring now to FIG. 8A, a biopsy sample 172' has been taken from the tissue cassette 100 of FIG. 1 and placed into a mold 170. After placing the biopsy sample 172' into the mold 170, the biopsy sample 172' is embedded in a material such as paraffin wax. Sections are then taken with a microtome, such as through line 178. The tissue cassette 100 of FIG. 1 has formed the biopsy sample 172' flat so the line 178 passes through the lesion 180'. FIG. 8B illustrates that the medical professional places the section taken through line 178 upon the slide 182 in preparation for diagnosis. The biopsy sample 172' has a flat section 172a' that is visible along the entire length of the biopsy sample 172'. Accordingly, the section of the lesion 180a' appears inside of the flat section 172a' and will be discovered by the pathologist or other medical professional. Thus, the tissue cassette 100 helps ensure that the proper diagnosis of a biopsy sample 172 is performed.

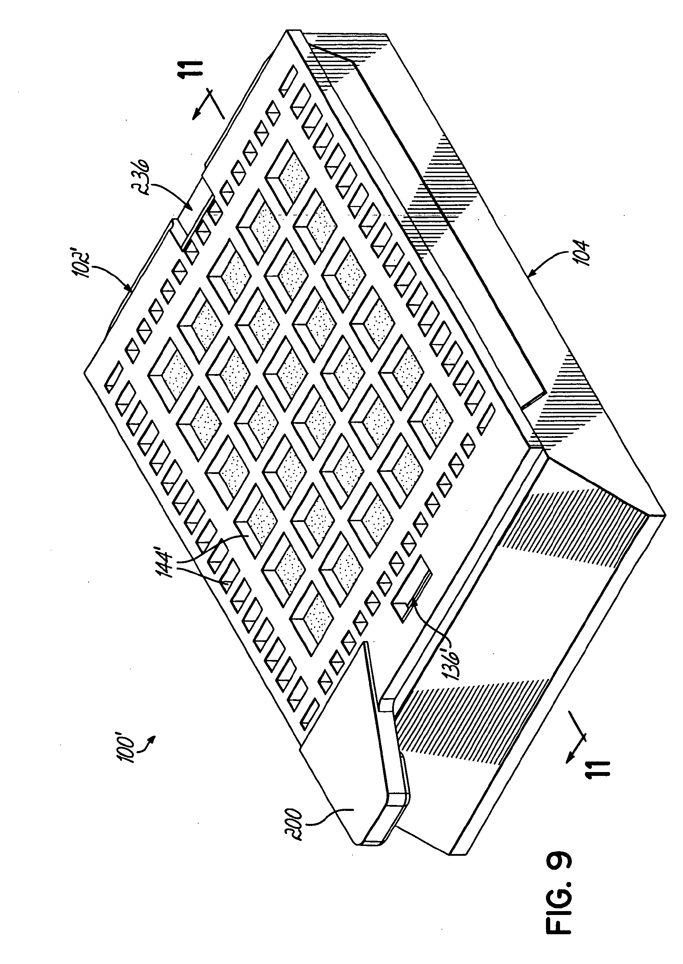

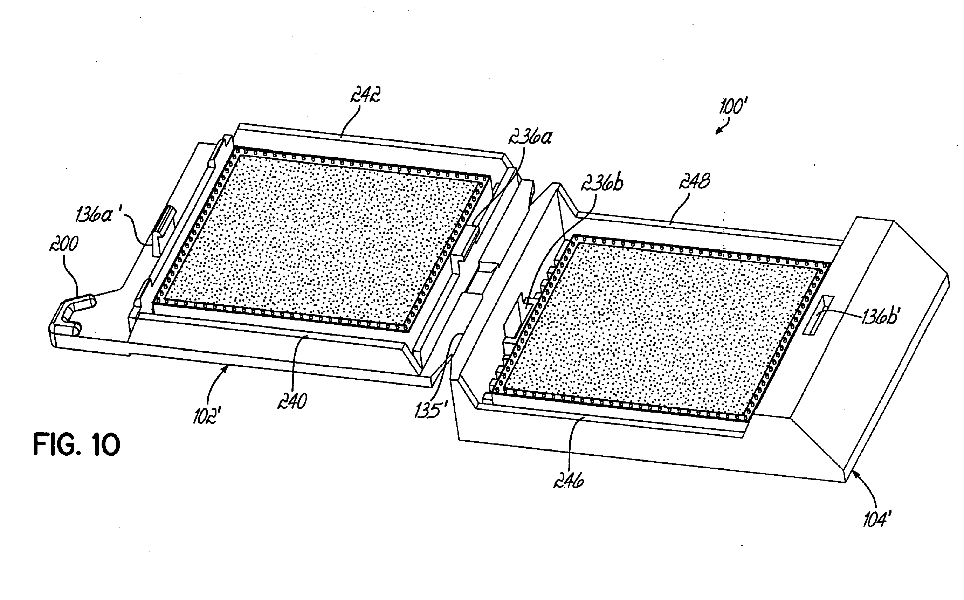

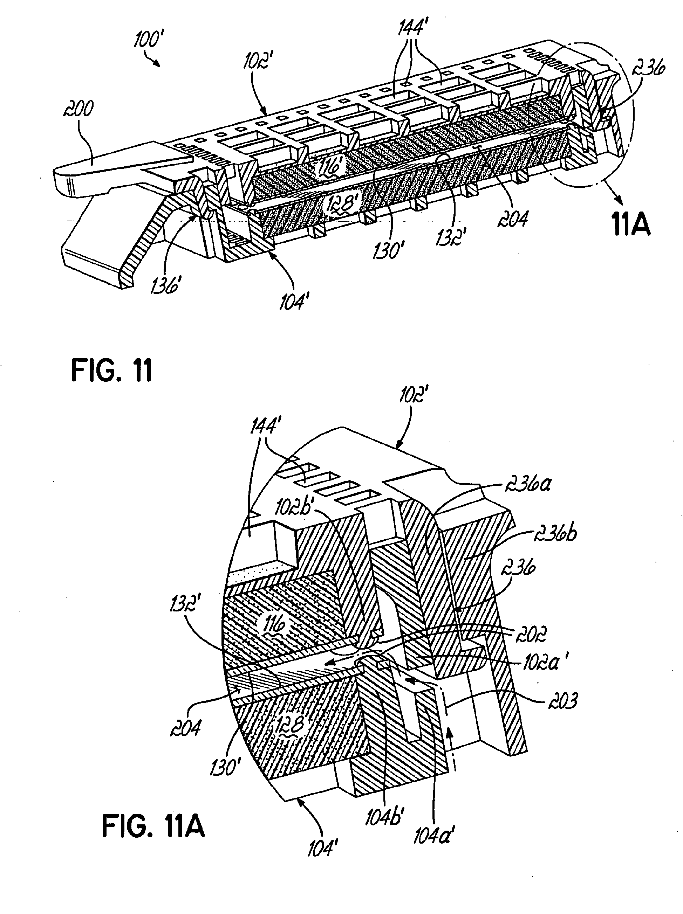

Referring now to FIGS. 9-11 and 11A, a tissue cassette 100' is shown according to a second illustrative embodiment. The tissue cassette 100' may include any of the features discussed above with respect to the first embodiment. Certain differences exist between the first and second embodiments as will be apparent from the following description and a review of the respective drawing figures. Like reference numerals with prime marks (') are used to illustrate corresponding elements in the first and second embodiments with structural and/or functional differences being apparent from a review of the respective drawing figures, the written description, or both. The tissue cassette 100' comprises first and second perforated structures, including a lid 102' and a base 104' which may be connected by a hinge 135' at one end and a clasp structure 136' at the opposite end. Apertures 144' are provided to allow fluid flow into the cassette 100'. The clasp structure may comprise a projection 136a' on the lid 102' received within a recess 136b' on the base 104'. The lid 102' also includes a second clasp structure 236 adjacent to the hinge 135'. This clasp structure 236 comprises a projection 236a and a recess 236b as best shown in FIG. 10 in the open position and FIG. 11A in the closed position. As the lid 102' is closed, the hinge, which is frangible, breaks and the clasp structure 236 engages to hold the lid 102' to the base 104' in the closed position along with clasp structure 136' as shown in FIGS. 11 and 11A. Sidewalls 240, 242 of the lid 102' are received within complementary receiving areas 246, 248 of the base 104' (FIG. 10). Therefore, in the closed position shown in FIG. 9, tissue samples are prevented from escaping from the sides of the cassette 100' as well as the ends of the cassette 100' having the respective clasp structures 136', 236. A tab 200 on the lid 102' may be used to apply upward force on the lid 102' to decouple the clasp 136'.

As shown in FIGS. 11 and 11A, porous membranes 130', 132' may be heat staked to the perforated structures 102', 104' through the use of projections 202. These projections 202 are shown in FIG. 10 prior to the heat staking operation as small cylindrical elements. After heat staking, the cylindrical elements 202 form mushroom-shaped heads which retain the edges of the membranes 130', 132' against the upper surfaces of the porous structures or foam 116', 128'. As further shown in FIGS. 11 and 11A, a fluid path may be formed completely between the first and second perforated structures 102', 104' such that fluid (such as formalin) may travel between the lid 102' and base 104' in the direction of the arrow 203 shown in FIG. 11A. The fluid path extends between additional portions 102a', 102b', 104a', 104b' of the perforated structures 102', 104' and between the "mushroomed" projections 202. Projections 202 may be staggered relative to each other when the lid 102' is closed instead of aligned as shown in FIG. 11A. This fluid path allows full saturation of the tissue sample(s). A spacing 204 is also shown between the upper and lower porous membranes 130', 132'. The spacing 204 may or may not be present depending on the needs of a particular application. For example, larger tissue samples may require a spacing 204 of any suitable dimension that will still allow the sample to be properly held, while smaller specimens may require no spacing 204.

It should further be noted that a nonstick coating may be applied to one or both of the porous membranes 130', 132' such that the tissue sample or samples may be easily removed from the surface 130' or 132'having a nonstick coating. Alternatively, the membrane material itself may comprise a nonstick-type material such as PTFE. As another alternative, one or more tissue samples may be adhesively secured on one of the porous membranes130' or 132' so as to retain the sample(s) on the membrane 130' or 132'. After fixing with a fluid such as formalin, the membrane having the adhesively secured tissue sample or samples may be cut out of the cassette 100' and placed in the bottom of an embedding mold or sectionable cassette for embedding in a material such as paraffin. Whether the tissue sample or samples are adhesively secured to one of the membranes or not, the lid 102' may be entirely removed from the base 104' and discarded either before or after the biopsy sample or samples are retrieved. The biopsy sample or samples may be placed in the bottom of a conventional paraffin mold and the mold may be filled with molten liquid paraffin. While the paraffin is still molten, the base 104' may be placed into contact with the paraffin. The paraffin then cools and hardens. The base 104' may then be used as a fixture for retention in a microtome chuck. A microtome operation may then be performed on the hardened paraffin and slide preparation and analysis may take place.

While these embodiments have been described in considerable detail, additional advantages and modifications will readily appear to those skilled in the art.

## Claims

1. A cassette for holding tissue comprising a first flat reference porous structure for supporting the tissue, a second porous structure having a compression resistance, wherein the first flat reference porous structure and the second porous structure can be positioned into an abutting position for securing the tissue therebetween.

2. The cassette of claim 1 further comprising a second perforated structure defining an interior area containing the second porous structure therebetween.

3. The cassette of claim 2 wherein a connector connects said first flat reference porous structure and said second perforated structure and compresses said structures together to firmly trap the tissue therebetween.

4. The cassette of claim 3 wherein said connector connects said first flat reference porous structure and said second perforated structure using a clasp and a hinge.

5. The cassette of any one of claims 2 to 4 wherein the first flat reference porous structure and said second perforated structure are arranged to form a clam shell type configuration.

6. The cassette of any preceding claim further comprising a first perforated structure defining an interior area containing the first flat reference porous structure having a second compression resistance.

7. The cassette of any preceding claim wherein said second porous structure includes foam to provide a predetermined compression resistance.

8. The cassette of any preceding claim wherein said second porous structure include a non-artifact inducing porous membrane.

9. The cassette of any preceding claim wherein the compression resistance of the first flat reference porous structure is greater than the compression resistance of the second porous structure.

10. The cassette of any preceding claim wherein one of the porous structures has a landmark indication system inscribed thereon.

11. A method for holding a tissue sample, comprising placing the biopsy sample into a tissue cassette, and moving at least a portion of the tissue cassette to apply a compressive force to flatten the biopsy sample against a flat reference surface of the tissue cassette.

12. The method of claim 11 wherein the tissue sample is placed into the tissue cassette on a landmark indication system corresponding to a location of harvest of the tissue sample.

13. The method of claim 12 further including marking a label on the landmark indication system corresponding to the location or orientation of harvest of the tissue sample from the patient.

14. The method of any one of claims 11 to 13 wherein moving the tissue cassette occurs by rotating a portion of the tissue cassette around a pivot.

15. The method of any one of claims 11 to 14 further comprising flattening the tissue sample between the flat reference surface and a compressive, porous structure.
